# EUROPEAN PATENT APPLICATION

(11) **EP 1 535 932 A1**
(43) Date of publication of application: **01.06.2005**
(21) Application number: 03764194.1
(22) Date of filing: 14.07.2003
(51) Int. Cl.: C08F 6/00, C08F 8/04, C08F 10/08

(54) **PROCESS FOR PRODUCING BUTENE OLIGOMER**

(30) Priority: 15.07.2002 JP 2002205598
(71) Applicant: IDEMITSU KOSAN COMPANY LIMITED, Tokyo 100-0005 (JP)
(72) Inventor: SARUWATARI, Tetsuya, Shunan-shi, Yamaguchi 745-0843 (JP); YAMANE, Hideki, Shunan-shi, Yamaguchi 745-0843 (JP); FUJITA, Toshiyasu, Shunan-shi, Yamaguchi 745-0843 (JP); NAKAMURA, Minoru, Ichihara-shi, Chiba 299-0107 (JP)
(74) Representative: Gille Hrabal Struck Neidlein Prop Roos
(86) International application number: PCT/JP2003/008923
(87) International publication number: WO 2004/007565

(57) **Abstract**

A process for producing a butene oligomer which comprises: polymerizing a butane/butene mixed fraction in the presence of an acid catalyst to yield oligomers; and then either (1) fractionating the oligomers by distillation to obtain a desired fraction and subjecting the fraction to hydrogenation and then deodorizing treatment or (2) hydrogenating the oligomers, subsequently fractionating the oligomers by distillation to obtain a desired fraction, and deodorizing the fractions. By this process, a butane oligomer which is odorless and has excellent oxidative stability can be produced.

## Description

### TECHNICAL FIELD

The present invention relates to a process for producing a butene oligomer. More particularly, it pertains to a process for efficiently producing a butene oligomer which is odorless and excellent in oxidative stability.

### BACKGROUND ART

In general, a butene oligomer is a liquid polymer which has an average molecular weight in the range of 150 to 2500, approximately and which is obtained by using as a starting raw material, a residual fraction (butane / butene mixed fraction) remaining non-extracted when butadiene is extracted from C₄ fraction formed by naphtha cracking.

The butene oligomer, which is excellent in electrical properties, stability, miscibility, water resistance and further permeation resistance against gases and steam, is widely used in applications of, for instance, electrical insulation materials, adhesives, lubricating oil bases, waterproofing agents, greases, viscosity index improvers, sealing compounds, wax, solvents and modifying agents for rubber and plastics.

As a process for producing the above-mentioned butene oligomer, a variety of production processes are known, including a process which comprises passing a starting raw material composed of a butane / butene fraction through slurry in which Friedel Crafts catalyst such as aluminum chloride anhydride is suspended; a process which comprises bringing the above-mentioned fraction into contact with a solid catalyst composed of dry chlorinated alumina {Japanese Patent Application Laid-Open No.82325 / 1982 (Show 57)}; a process which comprises bringing the above-mentioned fraction into contact with a solid catalyst such as fluorinated alumina, alumina-boron or silica-alumina {Japanese Patent Application Laid-Open No.40618 / 1981 (Show 56)}; and a process which comprises oligomerizing isobutylene by the use of a fired product of silica-alumina-zinc oxide or hydrogen type crystalline aluminosilicate {Japanese Patent Application Laid-Open Nos.79629/ 1981 (Show 56) and 108023 / 1982 (Show 57)}. In addition from an industrial aspect, a process of standard system and a process of Cosden system are put into practical application.

However, the butene oligomer which is obtained by any of the above-cited processes suffers from the disadvantage of inferior oxidation-stability, since the oligomer usually has strong odor and besides an olefinical double bond in its molecule. For this reason, improvements on the odor and oxidation stability have been strongly desired. With regard the odor, there is disclosed the use of for instance, a silica-alumina based adsorbent for the purpose of deodorizing {refer to Japanese Patent Application Laid-Open No. 124602/ 1985 (Show 60)}. However, the oxidation stability, which is insufficient in this case, results in deterioration of odor due to an ester which is formed by oxidation reaction at the time of storage.

### DISCLOSURE OF THE INVENTION

It is an object of the present invention to provide under such circumstances, a process for efficiently producing a butene oligomer which is odorless and excellent in oxidative stability.

It being so, intensive extensive research and investigation were accumulated by the present inventors in order to achieve the above-mentioned object. As a result it has been found that the object can be achieved by a process which comprises the steps of distilling an oligomer which has been obtained by polymerizing a butane-butene mixed fraction, separately collecting a required fraction, subsequently hydrogenating the same, and thereafter subjecting the hydrogenated product to a deodorizing treatment; or by a process which comprises the steps of hydrogenating an oligomer which has been obtained by polymerizing a butane-butene mixed fraction, subsequently distilling the same, separately collecting a required fraction, and thereafter subjecting the fraction to a deodorizing treatment. The present invention has been accomplished by the aforesaid findings and information.

Specifically the present invention provides;
(1) a process for producing a butene oligomer which comprises polymerizing a butane-butene mixed fraction in the presence of an acid catalyst to yield oligomers, thereafter fractionating the resultant oligomers by distillation to obtain a required fraction, subsequently hydrogenating the same, and thereafter subjecting the hydrogenated product to a deodorizing treatment;
(2) a process for producing a butene oligomer which comprises polymerizing a butane-butene mixed fraction in the presence of an acid catalyst to yield oligomers, thereafter hydrogenating the resultant oligomers, then fractionating the hydrogenated product by distillation to obtain a required fraction, and subsequently subjecting the resultant fraction to a deodorizing treatment; and
(3) the process for producing a butene oligomer as set forth in the item (1) or (2) wherein the oligomers after the hydrogenation has a bromine number of at most 5 g / 100 g and an aromatic component concentration of at most 100 ppm.

### THE MOST PREFERRED EMBODIMENT TO CARRY OUT THE INVENTION

In the process for producing a butene oligomer according to the present invention, a butane-butene mixed fraction is used as a starting raw material, and there are consecutively put into practice the steps of polymerization, fractionation, hydrogenation and deodorizing treatment or the steps of polymerization, hydrogenation, fractionation and deodorizing treatment. In what follows, some description will be given of each of the aforesaid steps.

### (1) Polymerization step

The polymerization step is the step of polymerizing a butane-butene mixed fraction as a starting raw material in the presence of an acid catalyst to yield oligomers.

There is usable as the butane-butene mixed fraction, residual fraction, which remains non-extracted when butadiene is extracted from C₄ fraction that is formed by naphtha cracking, which is called a spent B-B fraction (butane-butene mixed fraction), and which has hitherto been industrially used in general.

In addition, the acid catalyst is not specifically limited, but may be properly optionally selected for use from previously well known catalysts that have heretofore been usable as those for the polymerization of butene. Examples of such catalysts include a Friedel Crafts catalyst and a solid catalyst. Examples of the Friedel Crafts catalyst include Lewis acids such as aluminum chloride, aluminum bromide, ferric chloride, boron trifluoride, stannic chloride and zinc chloride; and strong protonic acids such as sulfuric acid and hydrofluoric acid. The above-mentioned Friedel Crafts catalyst may be used alone or in combination with at least one other species. Of these, aluminum chloride is particularly preferable.

On the other hand, examples of the solid catalysts include silica-alumina, silica-magnesia, silica-boria, alumina boria, chlorinated alumina, fluorinated alumina, silica-gel and alumina-gel, each being incorporated through adhesion with hydrochloric acid, sulfuric acid, phosphoric acid, BF₃ or the like; cation -exchange resin; synthetic zeolite; and further, clay minerals such as acid clay, bentonite, kaolin and montmorillonite. The above-mentioned solid catalyst may be used alone or in combination with at least one other species. Of these, silica-alumina is particularly preferable.

With regard to polymerization conditions, in the case where the foregoing Friedel Crafts catalyst is used, there is generally adopted liquid polymerization process, wherein the reaction temperature is selected in the range of usually 0 to 150°C, preferably 100 to 130°C, and the reaction pressure is selected in the range of usually 0 to 3 MPa·G, preferably 1.5 to 3 MPa·G. The polymerization system may be either batch-wise or continuous system.

On the other hand, in the case where the foregoing solid catalyst is used, the reaction temperature is selected in the range of usually 20 to 180°C, preferably 50 to 150°C, and the reaction pressure is selected in the range of usually atmospheric pressure to 10 MPa·G, preferably such pressure as being capable of maintaining a liquid phase (1 to 6 MPa·G , approximately). In the case of liquid-phase reaction, an LHSV (liquid hourly space velocity) of the starting raw material is selected in the range of usually 0.01 to 50 h⁻¹, preferably 0.1 to 10 h⁻¹. As the polymerization system, there may be adopted a continuous flow system in which a starting raw material is fed in a catalyst-packed tower.

Liquid oligomers which have an average molecular weight of about 150 to 2500 are formed by the above-described polymerization reaction.

### (2) Fractionation step

The fractionation step is the step of collecting required fraction by distillationally treating the polymerization reaction liquid which has been obtained in the preceding polymerization step.

In the first place, unreacted C₄ fractions are removed from the above-mentioned polymerization reaction liquid by means of distillation. Subsequently the reaction liquid from which the C₄ fractions have been removed is distilled to fractionate the same in accordance with boiling point ranges. The fractionating method is optional, and exemplified, for instance, by separation into a fraction having a boiling point of lower than 160°C, a fraction having a boiling point of from 160°C to lower than 200°C, a fraction having a boiling point of from 200°C to lower than 280°C and a fraction having a boiling point of 280°C or higher.

### (3) Hydrogenation step

The hydrogenation step is the step of hydrogenationally treating the required fractions which have been obtained in the preceding fractionation step to remove olefinical double bonds in the molecules and aromatic rings therein.

With regard to the above-mentioned required fractions, optional fractions may be properly optionally selected from among each of the fractions in the fractionation step in accordance with the purpose of use of the butene oligomer that is the finally obtainable product.

The catalyst which is used in the hydrogenation reaction is not specifically limited provided that it has hydrogenating activities for olefins and aromatic compounds, and may be used by properly optionally selecting from previously well known hydrogenation catalysts. Preferable examples of such hydrogenation catalysts include for instance, 0.05 to 0.5% by mass of Pd supported on alumina; 0.1 to 1 % by mass of Pd and 0.1 to 1 % by mass of Pt that are supported on alumina; zeolite catalyst and Ni / diatomaceous earth catalyst.

The hydrogenation reaction conditions include a reaction temperature in the range of 150 to 300°C, approximately, a reaction pressure in the range of 1 to 5 MPa·G, approximately and LHSV in the range of 0.1 to 10 h⁻¹, approximately. Preferably, the hydrogenation reaction is put into practice so that the oligomers after the hydrogenation has a bromine number of at most 5 g/ 100 g and an aromatic component concentration of at most 100 ppm.

The bromine number and an aromatic component concentration as mentioned above are values that are measured by the method described as follows.
Bromine number: 1 to 10 g of a sample which has been dissolved in a solvent is titrated at room temperature with a standard solution of potassium bromide / potassium bromate. The end point of the titration is regarded as the point of time when the polarization voltage in the titration system is rapidly altered by free bromine, while the point is detected with an electrical titration apparatus.
Aromatic component concentration: a sample to be measured or dilute solution thereof is placed in a quartz cell having an optical path length of 10 mm, and difference in absorbance from a blank at 270 mm is measured as a measured value by the use of a spectrophotometer.

In the present invention, the above-mentioned hydrogenation step may be conducted prior to the aforesaid fractionation step.

### (4) Deodorizing treatment step

The deodorizing treatment step is the step of deodorizingly treating the hydrogenated oligomers which have been obtained in the above-mentioned hydrogenation step or the required fraction (hydrogenationally treated oligomers) which have been obtained in the fractionation step.

The deodorizing treatment method is not specifically limited, but can be carried out, for instance, by a method in which the hydrogenated oligomers are brought into contact with an adsorbent of silica-alumina base or the like. More specifically, the oligomers are subjected to deodorizing treatment by feeding the oligomer into a deodorizing column packed inside with the aforesaid adsorbent under the conditions including a temperature in the range of 20 to 50°C, approximately and a LHSV in the range of 0.1 to 10 h⁻¹, approximately.

In such a manner, there is obtainable butene oligomers that are odorless and besides, excellent in oxidative stability.

In what follows, the present invention will be described in more detail with reference to comparative example and working examples, which however shall not limit at all the present invention thereto.

In the following comparative example and working examples, a bromine number and an aromatic component concentration were measured by the method described hereinbefore. The evaluation of the odor was made by 30 panelists through functional tests.

### [Example 1]

A mixed starting raw material composed of butane and butene at a mass ratio of 10 : 90 was polymerized under the conditions including a temperature of 120°C, a pressure of 2 MPa·G, a reaction time of 0.5 hour and the presence of 0.3% by mass of aluminum chloride catalyst, so that butene oligomers were obtained.

Subsequently the above-obtained butene oligomers were subjected to hydrogenation treatment under the conditions including a temperature of 200°C, hydrogen pressure of 2.5 MPa·G, a reaction time of 3 hours and the presence of a catalyst composed of 0.5% by mass of Pd supported on alumina in an amount of 10% by mass based on the butene oligomer. The resultant butene oligomer after the hydrogenation treatment had a bromine number of 0.5 g / 100 g and an aromatic component concentration of 60 ppm.

Subsequently the resultant hydrogenation reaction liquid was separated into a fraction having a boiling point of lower than 160°C, a fraction having a boiling point of from 160°C to lower than 200°C, a fraction having a boiling point of from 200°C to lower than 280°C and a fraction having a boiling point of 280°C or higher. The oligomers having a boiling point of from 200°C to lower than 280°C among them were subjected to a deodorizing treatment by passing the same through a glass made column packed inside with 100 milliliter of a deodorant available on the market (manufactured by Mizusawa Chemical Co., Ltd. under the trade name "GB08") at ordinary temperature, atmospheric pressure and a flow rate of 100 milliliter / hour. An evaluation was made of the odor of the oligomer thus subjected to the deodorizing treatment. As a result, they were evaluated as being odorless by all the panelists.

### [Comparative Example 1]

The procedure in Example 1 was repeated to evaluate the odor of the oligomers having a boiling point of from 200°C to lower than 280°C except that the hydrogenation and deodorizing treatments were omitted . A valuation was made of the odor of the oligomer thus subjected to the deodorizing treatment. As a result, they were evaluated as being odorous by all the panelists.

### [Example 2]

The oligomers having a boiling point of from 200°C to lower than 280°C which were obtained in Example 1 and subjected to deodorizing treatment were stored in the air for a period of one month, and thereafter evaluated for odor. As a result, they were evaluated as being almost odorless by all the panelists.

### INDUSTRIAL APPLICABILITY

It is made possible by the process according to the present invention to efficiently obtain butene oligomers that are odorless and excellent in oxidation stability from butane / butene mixed fractions as starting raw materials. Thus the butene oligomers that are produced by the process according to the present invention are preferably suitably usable for electrical insulation materials, adhesives, lubricating oil bases, waterproofing agents, greases, viscosity index improvers, sealing compounds, wax, solvents, modifying agents for rubber and plastics and the like.

## Claims

1. A process for producing a butene oligomer which comprises polymerizing a butane-butene mixed fraction in the presence of an acid catalyst to yield oligomers, thereafter fractionating the resultant oligomers by distillation to obtain a required fraction, subsequently hydrogenating the same, and thereafter subjecting the hydrogenated product to a deodorizing treatment.

2. A process for producing a butene oligomer which comprises polymerizing a butane-butene mixed fraction in the presence of an acid catalyst to yield oligomers, thereafter hydrogenating the resultant oligomers, then fractionating the hydrogenated product by distillation to obtain a required fraction, and subsequently subjecting the resultant fraction to a deodorizing treatment.

3. The process for producing a butene oligomer according to Claim 1 or 2 wherein the oligomers after the hydrogenation has a bromine number of at most 5 g / 100 g and an aromatic component concentration of at most 100 ppm.
